# EUROPEAN PATENT SPECIFICATION

(11) **EP 3 545 908 B1**
(45) Date of publication and mention of the grant of the patent: **09.12.2020**
(21) Application number: 18164627.4
(22) Date of filing: 28.03.2018
(51) Int. Cl.: A61F 2/30, A61C 8/00, B42D 25/30, G02B 5/02

(54) **MEDICAL PROSTHESES, MEDICAL OSTEOSYNTHETIC DEVICES OR HEARING AIDS WITH SECURITY AND/OR IDENTIFICATION ELEMENTS**
MEDIZINISCHE PROTHESEN, MEDIZINISCHE OSTEOSYNTHESEVORRICHTUNGEN ODER HÖRGERÄTE MIT SICHERHEITS- UND/ODER IDENTIFIKATIONSELEMENTEN
PROTHÈSES MÉDICALES, DISPOSITIFS MÉDICAUX OSTÉOSYNTHÉTIQUES OU PROTHÈSES AUDITIVES COMPORTANT DES ÉLÉMENTS DE SÉCURITÉ ET/OU D'IDENTIFICATION

(43) Date of publication of application: 02.10.2019
(73) Proprietor: CSEM Centre Suisse d'Electronique et de Microtechnique SA - Recherche et Développement, 2002 Neuchâtel (CH)
(72) Inventor: ESTERMANN, Marcel, 3293 Dotzigen (CH); WASER, Jasmin, 4466 Ormalingen (CH); SCHNEIDER, Christian, 4665 Oftringen (CH); LUU-DINH, Angélique, 68100 Mulhouse (FR); SCHNIEPER, Marc, 1213 Onex (CH); KALLWEIT, David, 79115 Freiburg im Breisgau (DE); KRÄHENBÜHL, Roger, 4144 Arlesheim (CH); DE WILD, Michael, 4103 Bottmingen (CH); MAREK, Romy Linda, 8124 Maur (CH)
(74) Representative: Bremi, Tobias Hans

(56) References cited:
- EP-A2- 1 733 704
- WO-A1-2004/045866
- DE-B3-102010 024 899

## Description

### TECHNICAL FIELD

The present invention relates to medical prostheses, medical osteosynthesis devices or hearing aids (including housings and parts thereof) which consist of or are essentially made of metal, at least in the main structural parts, typically of stainless steel or a titanium alloy as used for such devices. Furthermore, the present invention relates to methods for making such devices.

### PRIOR ART

The number of placed dental implants, endoprostheses for hip and knee is increasing year by year worldwide. MedTec products are becoming safer and long-term success rates are improving. The market pressure forces lower healthcare expenses, reduced therapy costs and finally reduced manufacturing costs.

Currently, dental prosthetics, mostly from premium suppliers, are copied and sold legally (e.g. NT trading). So called copycats as well as newly founded implant producers start coping established prosthetic products by reengineering the implant connection. This development is unfavorable for the patient, since reengineered products do not meet the exact specifications of the engineered design, often do not meet the quality standards and regularly failures are reported. On the other hand, the market for copycats is growing fast due to lower prices. Lower prices are mainly determined by a weaker quality system and the lack of clinical studies. The influence of CADCAM solutions is likely to speed up this development quickly.

Responsible users ask for original parts. However, it is difficult for a surgeon to find out if an original or a copied prosthetic part was used, since this decision which product to use is made at the dental technicians site and is not visible on the part. It is assumed that the amount of all prosthetic parts purchased through copycats will shift to 50% within the next 5 years in Europe and US. This is equivalent to 40% of the complete prosthetic sales. Furthermore, it is assumed that 50% of the surgeons would preferably use original parts, if they could distinguish between. Therefore, a 20% loss of sales of prosthetic products is expected, if no identification of the original manufacturer will be applied.

A very important further topic is guaranty claims for damaged parts. Guaranty claims have to be investigated carefully, to assure original parts have been used. One has observed an increasing use of copied pieces over the last two years.

Copied medical implants are not only found in the dental industry, but the entire medical industry.

Patent document EP 1733704 discloses orthopaedic implants provided with a superparamagnetic material in the form of a symbol, pattern or another type of indicia in order to determine the position and/or wear of the implant itself.

### SUMMARY OF THE INVENTION

Many attempts have been made to avoid copying by track and tracing of medical devices. These attempts include packaging with elaborate security features, but also laser marking of the medical devices themselves or attaching security elements to the medical devices. The problem with the former is that after the medical device has been unpacked there is no possibility to check the identity and/or origin of the device anymore. The problem with the latter is that laser markings are conventional art and can be as easily reproduced as the medical device itself. Attaching security elements to medical devices is a problem since on the one hand as a rule these security elements will have to be removed before use of the implant, and attaching them to the implant requires adhesives which in the medical field are not tolerated.

The invention solves this problem by providing a device as claimed in claim 1, i.e. in the form of a medical implant or prosthesis, medical osteosynthesis device or hearing aid or hearing aid housing at least in part or essentially made of metal, which device comprises at least one optical security feature made with surface nano- and/or microstructuring, for example comprising a grating, typically a periodic submicron grating, which is directly embossed in an exposed metal surface in the form of a security and/or identification element.

Microstructuring is defined as the creation of surface structures which are submicronic in dimensions (i.e. periodicity smaller than 1 µm, typically in the range of 200-800 nm), in the micron-scale or of a few microns (typically at most 5 µm or at most 2 µm) in dimensions. For example for periodic nano- and/or microstructures such as gratings, the ridge/groove sizes can be submicronic (i.e. smaller than 1 µm, typically in the range of 200-800 nm) or up to a few microns (typically at most 10 µm or at most 5 µm), the depth of the microstructure in the submicron domain while the microstructure periodicity can be larger than one micron, for example less than or 2 µm.

The nano- and/or microstructures can comprise or consist of non-periodic nano- and/or microstructures such as Fourier or Fresnel Diffractive Optical Element (DOE), random microstructures, Optically Variable Devices (OVDs), Diffractive Optical Variable Image Devices (DOVIDs), micro-images, micro-structures encoding an image, code or symbol based on a Moire encoding, diffusive and scattering optical microstructures, zero-order color-generating optical microstructures and a combination thereof.

As is well known, medical prostheses, medical osteosynthesis devices or hearing aids made of metal make use of very hard metals or metal alloys. Surprisingly, it was found out that it is possible to emboss even the above-mentioned (grating) structures into exposed metal surface areas of these devices. The (grating) structures can be structured as topologies providing for variable impression depending on viewing angle (OVD) or even as holographic elements. Unexpectedly, it was found that it is possible by using corresponding metal stamps having the respective negative (complementary) topology on the stamping surface to generate plastic deformation in the corresponding metal surface so that a (grating) nano- and/or microstructure can be generated which, also under normal viewing conditions of the medical staff, can be recognized. Furthermore, the corresponding surface topology on the medical device has no negative impact on sterilize ability or healing in properties, and it is lasting on the implant or other medical device and can be verified, if for example the implant has to be removed from the patient.

The implant as such is typically essentially made completely of metal, it may however also comprise parts or sections which are made from a different material, so for example in the interior of the interface to an abutment a dental implant may comprise inserts made of a plastic material or the like. However, what is important is that the structural part of the device, so the load bearing part thereof, is essentially made of or consists of metal, and that the nano- and/or microstructure (grating) is embossed in that part in a region where metal is exposed. Another possibility is that the medical device in portions where the nano- and/or microstructure for generating the optical effect is not located, is provided with surface coatings. In case of implants it's for example possible that in the threading portion there is not only a rough surface with a tailored surface topology for improved healing and, but that there is an additional coating for improved osseo integration. So if mention is made of the device essentially consisting of metal this does not exclude such coatings and additional non-loadbearing components being made of a different material. The fact that the security feature is provided in the metal structural load-bearing component is another significant advantage since it is not easily possible to remove the security element in the form of the nano- and/or microstructure if it is provided in the structural part of the device itself.

According to a preferred embodiment, typically the metal of the device is selected from steel, preferably stainless steel or implant steel, or titanium or a titanium alloy with at least one of zinc, niobium, tantalum, vanadium, aluminium. For example systems of the type TiAl6Nb7 or TiAl6V4 are possible.

It was shown by a series of experiments that to be actually able to emboss a corresponding topology pattern into the surface of a metal, the periods of the one or more gratings being part of the security feature are preferably in the range of 0.3-3 µm, preferably in the range of 0.5 - 2 µm and more preferably in the range of 1-1.9 µm. Particularly good results in terms of embossing of the full area and depth of the generated grating could be achieved if the period of the gratings was chosen to be in the range of 1.7-1.9 µm.

Preferably, the depths of the embossed gratings part of the security feature are in the range of 80-500nm, preferably in the range 200-400 nm, and more preferably in the range of 230-300 nm.

The gratings and other possible nano- and microstructures can be embossed on a ground exposed metal part of the device.

Surprisingly, it was furthermore found that it is not mandatory that the exposed metal surface is of a particularly low surface roughness prior to the embossing process. As a matter of fact, it seems that if the parameters of the metal stamp used for embossing and of the process of embossing are properly chosen, a certain degree of surface roughness can be compensated due to the ductility of the metal and the flow of the metal during the embossing process. As a matter of fact, preferably the nano- and/or microstructure is embossed on an exposed metal part of the device having a surface roughness Ra (as defined according to ISO 4287:1997) of at most 0.8 µm, preferably of at most 0.5 µm or at most 0.3 µm or at most 0.23 µm, preferably in the range of 0.20-0.25 µm. Surprisingly it is possible to start with a surface roughness of the exposed surface that range.

The security feature is typically embossed using an embossing pressure in the range of 0.1-5 kN/mm², preferably in the range of 0.1 - 2 or 0.2-1 kN/mm². The embossing surface area is typically in the range of 3-5 mm². Also larger areas up to 5 cm² are possible.

Embossing at particularly elevated temperatures should be avoided since these can change the geometry and/or surface properties of the corresponding devices. On the other hand embossing of the nano- and/or microstructure, preferably the security feature comprising one or more gratings, needs to take place essentially at the end of the manufacturing process, for medical devices typically before sterilization and packaging. Surprisingly it was found that the nano- and/or microstructure, preferably the grating, can be embossed at comparably low temperatures, so preferably at a temperature of at most 150°C, preferably at most 100°C, preferably in the range of 10-40°C.

The device is preferably a dental implant and further preferably the nano- and/or microstructure, preferably the security feature comprising one or more gratings, is provided in the form of a patch in the coronal collar region of the dental implant, preferably on a bright finished metal portion thereof. Further preferably the one or more nano- and/or microstructures, preferably the gratings, are provided on an axial surface of the dental implant which after implantation and attachment of the abutment is covered by the abutment mounted on the implant. Alternatively or additionally the optical diffractive element can be provided on a bright finished exposed metal cylindrical or conical apical portion of the collar region.

The device can also be a dental abutment. Dental abutments are particularly prone to copying, since for the implants there are many patent protected designs and coatings and the like which have a tremendous impact on healing in properties and primary stabilization. On the other hand corresponding abutments are often standard devices and are not directly patent protected, so their use cannot be prevented. On the other hand very often these copied abutments do not meet the quality as well as size specification requirements in particular as concerns the interface to the implant. If therefore a non-original manufacturer abutment is combined with an original implant this not only causes problems during the mounting process of the abutment on the original implant it also causes problems later on if there is a problem with the combination of the implant and the abutment and then it cannot be found out easily anymore whether an original abutment was mounted on the original implant. It is therefore of particular importance that also abutments can be made more identifiable. Correspondingly, according to another preferred embodiment, wherein the nano- and/or microstructure, preferably the security feature comprising one or more gratings, is provided on a cylindrical or conical or a flattened portion of the protruding portion of the abutment. However also here it is possible to provide the one or more nano- and/or microstructure, preferably the gratings, on an axial surface which, once the abutment is mounted, is contacting a corresponding complementary axial surface on the implant.

The nano- and/or microstructure, preferably the security feature comprising one or more gratings, can be provided in the form of a patch with a surface area of at most 50 or at most 10 or at most 5 mm², preferably in the range of 2-4.5 mm².

The nano- and/or microstructure, preferably the security feature comprising one or more gratings, can be provided such that the tips of the one or more structures/gratings are essentially flush with the surface plane defined by the surrounding metal surface. Unexpectedly it was found that plastic deformation during the embossing process is sufficient that the embossed region is not recessed significantly with respect to the surrounding surface, by less than 40 microns, preferably by less than 20 microns. This is important in particular for the corresponding functional surfaces of the devices, which very often do not tolerate recessed portions for stability reasons and/or avoiding cavities fostering inflammations and the like.

The optical diffractive element comprising one or more structures/gratings typically and preferably generates the image of a picture and/or letters and/or numbers and/or pictograms and/or logos. The corresponding optical information can preferably be read out by the naked eye under conventional lightning conditions. It is however also possible that, alternatively or additionally, the corresponding optical information can be read out by a specifically tailored device. What is furthermore possible is that the information includes, apart from verification information easily recognizable by the end-user additional partly hidden information only recognizable by either using specific devices and/or algorithms as an additional level security feature and for tracking for example batches of manufacturing. Furthermore the present invention relates to a method for making such a device in the form of a medical prosthesis, a medical osteosynthesis device or a hearing aid or hearing aid housing, essentially made of metal, wherein the device comprises at least one nano- and/or microstructure which is directly embossed in an exposed metal surface in the form of a security and/or identification element.

Said method for producing a nano- and/or microstructure, preferably an optical diffractive element in the form of a grating on such a device is characterized in that a metal stamp carrying a topologically structured surface being essentially the negative of the nano- and/or microstructure (preferably the grating) to be generated on the device is embossed on an exposed metal surface of the device preferably under plastic deformation conditions such that the topology of the topologically structured surface is imaged on the metal surface of the device.

Preferably the metal stamp used has a grating depth in the range of 80-500 nm.

Further preferably the metal stamp at least in the region of the topologically structured surface for embossing consists of material of a higher hardness than the material of the device in the exposed region to be embossed.

Preferably the metal stamp is essentially based on hardened steel, preferably selected from the following steels: 1.2083, 1.2363, UM20 HIP, UM30 HIP, K110/1.2379, K340, K470, K890, Stavax ESR or ESU, Rigor 1.2363, Böhler K305, EN 1.2344, SKD61 1.2344, EN 1.2343, EN 1.2083, EN 1.2162, EN 1.2516, or RAMAX, or hardened steel with a hard coating, preferably with a coating of tungsten carbide, Si3N4 or ZrO2, cemented carbide such as tungsten carbide (WC), titanium carbide (TiC), or tantalum carbide (TaC) as the aggregate. Mentions of "carbide" or "tungsten carbide" in industrial contexts usually refer to these cemented composites. Such a hard coating can also be made of Cr Nitrides CrN or CrAIN, TiN, Diamond Like Carbon (DLC) or other suitable materials.

The nano- and/or microstructure (preferably in the form of a grating) is embossed using an embossing pressure in the range of 0.2-5 kN/mm2, preferably in the range 0.5-2kN/mm2. The nano- and/or microstructure can be embossed at a temperature of at most 150°C, preferably at most 100°C, preferably in the range of 10-40°C.

Last but not least the proposed invention relates to the use of a method as given above for making a device as given above identifiable and/or for providing it with a security element and/or marking.

Further embodiments of the invention are laid down in the dependent claims.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,

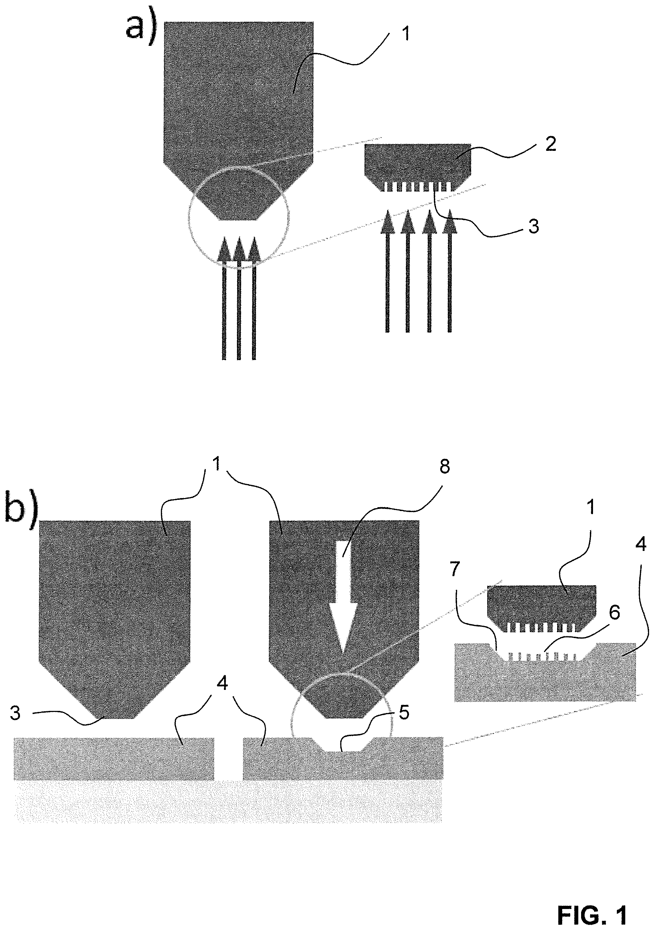

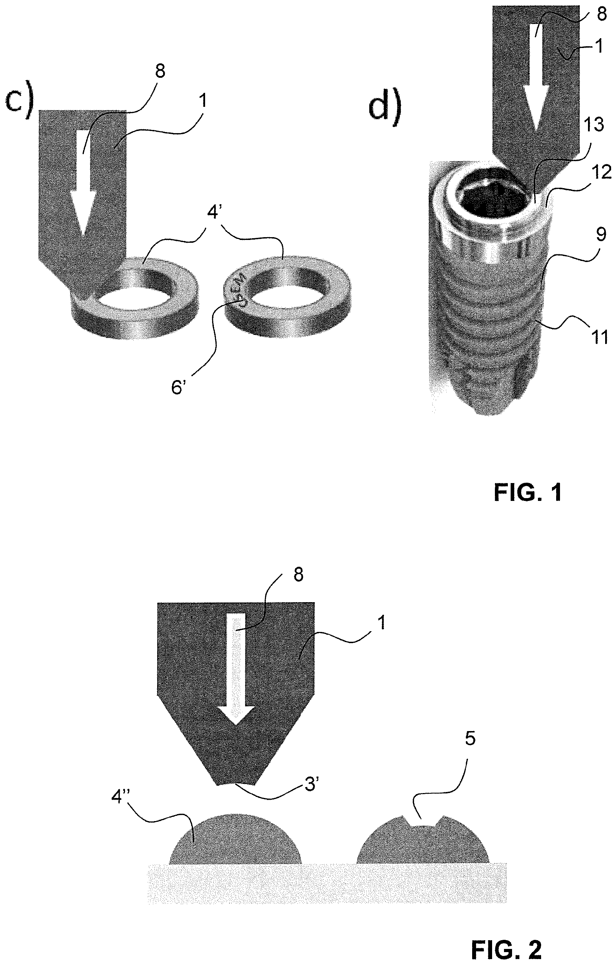

- Fig. 1: shows in
a) a metal stamp for use in the context of the present invention as well as in a magnified representation to the right the corresponding topologically structured surface region for embossing, in
b) on the left side the metal stamp and the element to be embossed before the step of embossing, in the middle the metal stamp and the element after embossing, and on the right side a magnified representation of the grating on the metal stamp and the embossing, in
c) on the left side the metal stamp and the element in the form of a ring to be embossed before the step of embossing, on the right side the embossed ring, and in
d) the embossing of a dental implant;
- Fig. 2: shows the situation of embossing a curved surface, wherein on the left side the situation before embossing, and on the right side the situation after embossing is schematically illustrated;
- Fig. 3: shows on the left side the embossing of a conical portion of the coronal collar of an implant and on the right side the embossing of a conical portion on an abutment;
- Fig. 4: shows in a) a laser scanning digital microscope image of the topologically structured surface on the metal stamp for an embodiment having a period of 1.8 µm, and in b) the properties along line 19 in a);
- Fig. 5: shows in a) a laser scanning digital microscope image of the resulting embossed grating on the object and in b) the properties along line 19 in a);
- Fig. 6: shows in a) a cut in a direction essentially perpendicular to the grating direction through an embossing generated in an abutment, in b) an image of an abutment with an optical diffractive element, in c) a REM image of the embossed grating and in d) the embossing region on the abutment;
- Fig. 7: shows a nanoimprinted microstructure on a flat steel stamp surface;
- Fig. 8: shows an opened nanoimprint material and open steel surface under AFM;
- Fig. 9: shows a simple diffractive transferred into hardened steel as seen under AFM;
- Fig. 10: shows a schematic representation of the metal stamp making process in schematic cut views showing the grating schematically as a zigzag pattern and the embossing process, wherein in a) the making of the soft stamp based on the master is shown, in b) the generation of the imprint based on the soft stamp is shown, in c) the attachment of the imprint on the metal stamp portion is shown, in d) the etch opened imprint on the metal stamp, in e) the front metal stamp portion after the metal etching and in f) the starting position for the embossing on the device to be embossed.

### DESCRIPTION OF PREFERRED EMBODIMENTS

The objectives of this invention is a microstructuring transfer/embossing process into for example a medical device, in particular into a titanium implant material of structures like holograms and Optical Diffractive Elements (DOE). This, to add optical security features consisting of nano - and/or microstructures, like sophisticated holograms, covert laser readable images, 2D/3D QR codes, logos, article or lot numbers or micro-text, directly into the titanium implant material such to create visual and appealing 1st level control security features on the one hand side, and/or to provide unique identifying (hidden) 2nd level control security features for trademark protection, e.g. to identify new or explanted fake implants and defeat product counterfeiting, on the other hand.

In the security world one usually defines 3 levels of security features:
First level are features visible by naked eyes and do not need any external set-up, typically holograms are 1st order security devices.

The second level features will need a simple external set-up, like a UV lamp, a laser pointer etc. easy to find on the market, UV inks and DOE's are often 2nd level security devices.

And the 3rd level are security features that can only be identified in the laboratory, like the real composition of a material, the measurement of traces of specific chemical compounds or elements.

Here, focus is put on first and second level security features.

Nano- and/or microstructure surface labelling is tissue-compatible because the process is only based on a pure physical structuring of the surface of the implant, and no chemicals, acids, paints, pigments, coatings or solvents need to be implemented.

Furthermore, the proposed markings are abrasion-resistant and that they can be disinfected and sterilized.

A new way of hologram tooling is used. As already mentioned this process permits to transfer complex holograms directly into hard steel surfaces. By illuminating a certain area on the micron-structured steel surface with a laser pointer, a logo and/or a data code is projected on a screen.

The steel nano- and/or microstructuring technology can be used to make high resistant stamping tools, capable to stamp the holograms into the titanium alloys those implants are made of.

This can be accomplished by using a stamping/embossing process after the production of the metal stamp.

Nano- and/or microstructuring is defined as the creation of surface structures which are submicronic in dimensions, in the micron-scale or of a few microns in dimensions. For example for periodic microstructures such as gratings, the ridge/groove sizes can be submicronic or up to a few microns, the depth of the microstructure in the submicron domain while the microstructure periodicity can be larger than one micron, for example 2 microns.

The making of the metal stamp 1 having a tip portion 2 with a topologically structured grating structure which is complementary to what is to be generated on the device is very schematically illustrated in figure 1 a).

Such a metal stamp can be generated using a technique as follows, using a new approach by directly transferring micro and nano-structures into a typically hardened steel material for the metal stamp. This increases drastically the stamp lifetime compared to conventional stamp making techniques. The structuration of 2D curved metal stamp surfaces with small radius of curvature is as well demonstrated. This technique and the resultant stamps allow the hot and cold embossing of various materials in very large volumes.

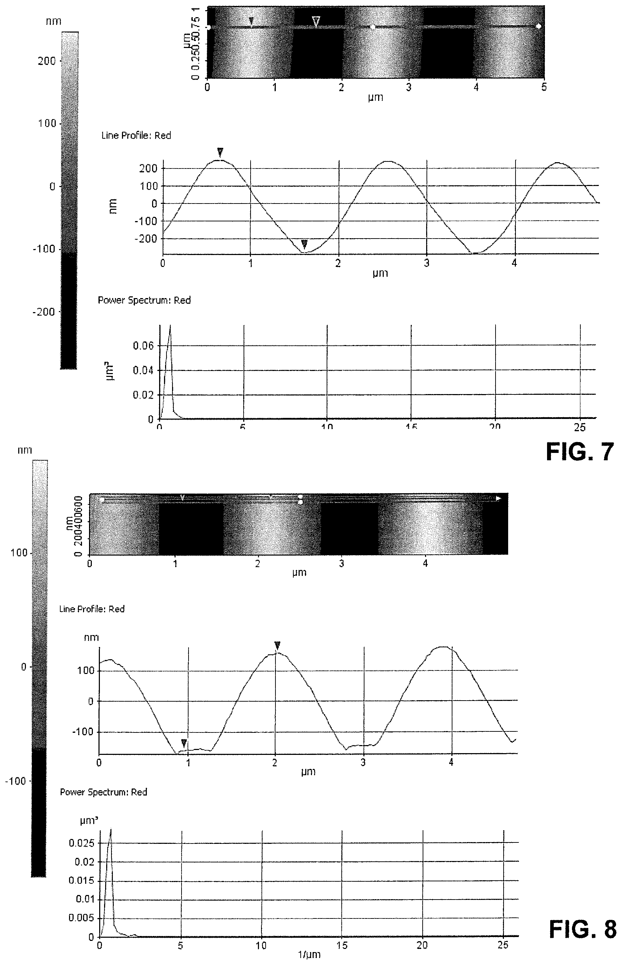

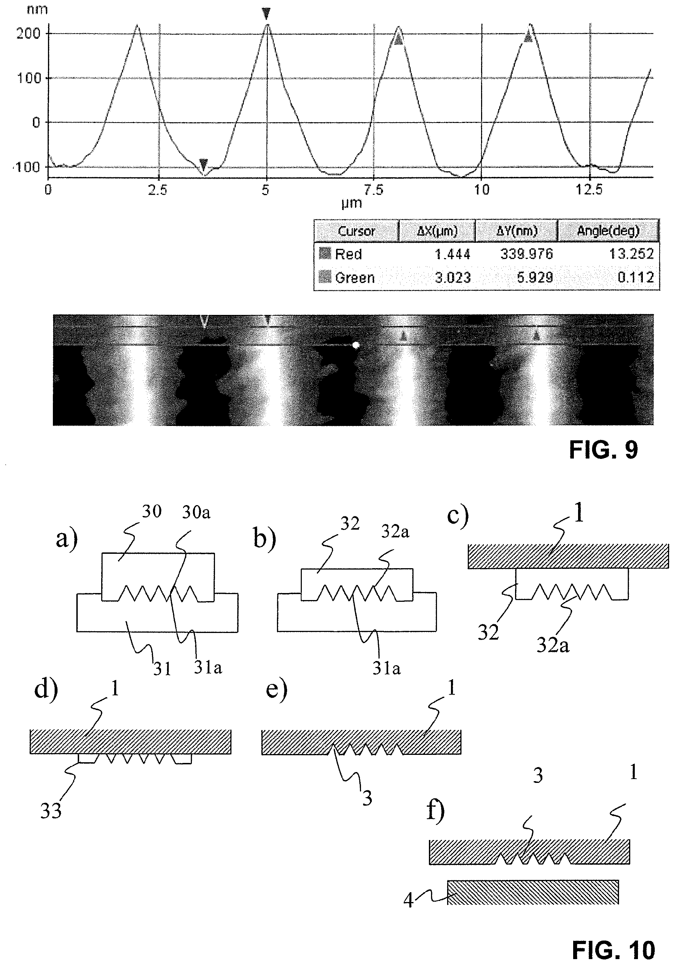

The structuration of steel or other metallic stamps relies on several process steps, some of which are optional depending of the tool and result to be achieved:
1. In a first step, the inner surface of the metal stamp, with which micro- and nanostructures should be embossed on the final part, should to be polished. Most steel grades have random rough surfaces in the scale to a few to several microns, in order to create a matt or dull finish on the polymer surfaces. To transfer successfully smaller structures with a high coverage, this topography needs to be planarized using polishing techniques. Various polishing technique, purely mechanical, purely chemical or combined mechanical and chemical etching can be used. The roughness target after this polishing step should be lower than the micro- or nanostructures to be transferred, in order get high surface coverage and optimal optical quality of the diffractive structures. Typically surface roughness, should be lower than (as defined according to ISO 4287:1997) 0.8 µm, preferably lower than 0.5 µm or lower than 0.3 µm or lower than 0.23 µm, preferably lower than 0.05 µm and more preferably as low as or lower than 0.020 µm. When possible and depending on the mold geometry, a so-called mirror finish polishing is preferable. Ultimately the grain size of the steel will limit the achievable planarization quality. To reach very low roughness levels, as may be interesting for the transfer of nanostructures, the use of cold-worked steels which have not be annealed is preferable. Useful for the present purpose of making metal stamps are steel types as follows: 1.2083; 1.2363; UM20 HIP; UM30 HIP; K110/1.2379; K340; K470; K890, Stavax ESR or ESU, Rigor 1.2363, Böhler K305, EN 1.2344, SKD61 1.2344, EN 1.2343, EN 1.2083, EN 1.2162, EN 1.2516, or RAMAX.
2. As a second step, a master tool containing the diffractive nano- and/or microstructures, whether simple grating or complex surface holograms, is replicated in a soft stamp material.
   Preferably the soft stamp material is a soft material allowing the soft stamp to be flexible. The master tool can be made of a photoresist material, a glass, a nickel shim, a fused silica master, a sol-gel replica or any other material depending of the origination, structure modification and structure assembly processes. A method how to produce such a master tool as well as possible materials for use is e.g. disclosed in Optical Document Security (R.L. Renesse, Optical Document Security, Third Edition, 3rd edition, Artech House, Boston, Mass., 2004). The soft stamp material is made of a flexible material, usually elastomeric, which is either hot embossed, UV embossed, UV casted, heat casted or heat and UV casted from the master tool.
   Possible specific materials for the soft stamp are as follows: silicon-based elastomers such as PDMS, urethane-based elastomer, polyurethane, polypropylene-based organic material, polyacrylates such as polymethyl-methacrylate (PMMA) or polycarbonate (PC), Polyester (PET), Polyamide (PA), a fluoropolymer such as ETFE or PTFE, polyimide (PI) and any combination thereof.
   If needed the flexible and usually elastomeric material can be casted or laminated on a flexible foil that will support it and limit its lateral deformation. Especially during the imprinting step, pressure can lead to stretching of the soft stamp material.
3. The third step consists in imprinting the structure transferred from the master tool through the soft stamp to the actual polished metal stamp surface. The imprint material is usually an acrylate based, preferably cross-linkable organic material. Possible specific materials for the imprint material are as follows: an acrylate based material (including methacrylate materials), a polyester-based material, an epoxy-based material or an urethane-based material, or mixtures thereof.
   The cross-linking of the imprint can be effected by UV exposure (UV induced cross-linking), a heating step (heat-induce cross-linking), UV and heat combined or using two-component cross-linkable materials.
   The imprint material is deposited either on the soft-stamp, for example using spin-coating or on the final metal stamp surface, for example using spray-coating.
   The final metal stamp surface is put it contact with the soft-stamp so that the imprint material located in between is pressed between the two materials. The pressure can be applied using a soft and deformable elastomeric tampon. The tampon geometry is usually adapted to the final metal stamp 3D shape to apply gradually a pressure for the soft-stamp center to its outer edges.
   After the cross-lining, the soft-stamp and metal stamp are demolded. To prevent damaging the imprint material or to delaminate the imprint material from the metal stamp surface, an anti-sticking agent can be applied on the soft-stamp before it comes into contact with the imprint material.
4. The imprint organic material transferred to the polished mold surface then needs to be etched. An AFM topography image of such a micro-structure can be seen in figure 7. The imprint material contains a continuous imprint material layer below its patterned upper surface.
   This residual layer is etch opened, usually using a dry technique method such a Reaction Ion Etching (RIE), preferably an oxygen based RIE. Suitable etching conditions are as follows: oxygen reactive ion beam etching of 4 minutes.
   This allows exposing back to air a portion of the steel polished surface, as can be seen in figure 8 under AFM.
5. The steel tool is now etched using Reactive Ion Beam Etching (RIBE) also called Ion Beam Milling (IBM), typically using ionized argon gas.
   Suitable etching conditions are as follows: Veeco RIBE plasma chamber with a duration of 25 minutes.
   Such dry etchings have relatively low selectivity between various metals. This allows the metal stamp tool, possibly made of hardened steel, to be etched in its bulk with relatively good depth as structure aspect ratio above 1 can be realized.
   At the end of this transfer, the imprint material and etching residues are either fully etched away or the residues left can be easily cleaned so that the metal stamp tool is back to its original composition. Figure 9 shows a simple diffractive transferred into hardened steel as seen under AFM.

The steps are schematically illustrated in figure 10.

Figure 10 a) shows the first step of the generation of the soft stamp. The master 30 is provided and its grating portion 30a is for example hot embossed into the soft stamp 31, so that in the corresponding surface of the soft stamp a replica of the grating portion 30a is generated forming the soft stamp grating 31a.

In the next step this soft stamp is used for making the imprint 32, this is illustrated in figure 10 b). To do so, for example in a casting process, imprint material is cast at least on the soft stamp grating portion 31a, so that the corresponding grating is again replicated forming the imprint grating 32a.

In the next step the result of which is illustrated in figure 10 c), the imprint is then transferred to the surface portion of the metal stamp 1 which shall be provided with the corresponding grating. It is also possible to directly form the imprint between the soft stamp 31 and the metal stamp 1, for example by providing the imprint material on the surface of the metal stamp 1 as a layer and then applying the soft stamp 32 to that coated surface portion.

In this phase the imprint 32 still is fully covering the corresponding area, the metallic surface of the metal stamp 1 not being exposed anywhere.

In the next step the imprint material is etch opened leading to the situation as illustrated in figure 10 d). The etch opened imprint 3D topologically structured surface 33 exposes now the corresponding metal portions as regular pattern.

In a following step, the result of which is illustrated in figure 10 e), metal etching takes place such that the open portions of the etch opened imprint 33 are etched a way leading to a corresponding grating in the surface of the metal stamp 1.

Now the metal stamp 1 or rather the corresponding topologically structured portion 3 thereof, can be used to emboss the corresponding optically active pattern in the corresponding device 4.

### A specific Example of a 1.2083 steel metal stamp production method is described for exemplary purpose:

In a first step, the surface of a metal stamp made of steel 1.2083, with which micro- and nanostructures should be embossed, is polished to be mirror-like. Most steel grades have random rough surfaces in the scale to a few to several microns, in order to create a matt or dull finish on the polymer surfaces. To transfer successfully smaller structures with a high coverage, this topography needs to be planarized using polishing techniques.

As a second step, a master tool containing the diffractive nano- and/or microstructures, whether simple grating or complex surface holograms is made of a nickel plate grown galvanically from a previous master, so-called a nickel shim. The nickel shim is coated with 10mL of a fluorinated and no-fluorinated acrylated/methacrylated mixture UV-Opti-Clad made by Ovation Polymers. The structured nickel shim coated with the mixture is pressed against a planar fused-silica wafer and flashed with 10W/cm of 365nm UV light. The cross-linked UV-Opti-Clad soft stamp is peel-off from the structured nickel shim and fused-silica wafer.
The structured surface of the soft stamp is activated with a 5 minutes thinned-air plasma in a Harrick PDC-32G plasma cleaner oven. A thermal imprint material is spin-coated on the activated structured surface at 2000 rotation per minute with a mr-I T85-5 imprint material from Micro-Resist Technology GmbH.

The third step consists in imprinting the structure transferred from the master tool through the soft stamp to the actual polished metal stamp surface. In order to press the imprint material on the metal surface, the backside of the soft stamp is pressed on with an elastomeric tampon with 50N/cm² using a pressing steel plate. The metal stamp is coated with, the imprint material, the soft stamp, the elastomeric tampon and the pressing steel plate is placed in an oven. The oven is heated up to 140°C for 2h.
The pressing steel plate, the elastomeric tampon and the soft stamp are removed during the cooldown, leaving the metal stamp surface coated with a thin imprint material layer structured with the opposite polarity of the soft stamp, having the same polarity as the nickel shim used.

The fourth step consists of an oxygen etching in a Veeco RIBE plasma chamber with the imprint material facing the plasma. The duration of the oxygen reactive ion beam etching is of 4 minutes to etch open the grooves of the structures to the metal stamp surface.
A second etching step is used to etch the micro- and nanostructures into the metal stamp using a Veeco RIBE plasma chamber with a duration of 25 minutes.

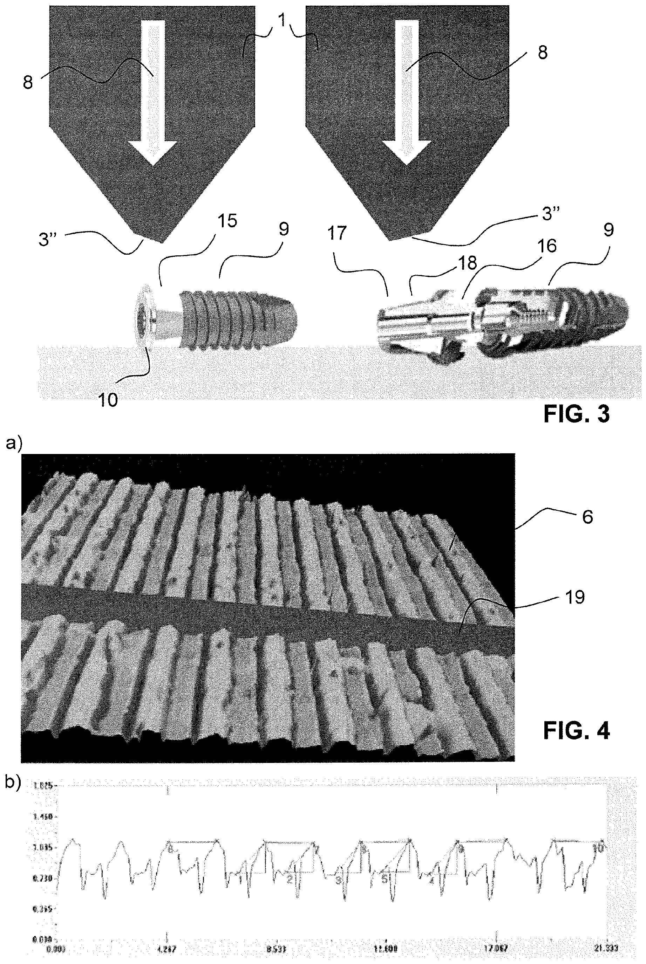

With the previously mentioned method, coated metal stamps can also be nano- and/or microstructured, for example by hard chrome electroplating. According to the method described above, a diffractive microstructure 3 is created in the surface.
If necessary, the metal stamp 1 or its surface 3 may be hardened after generating the microstructure by a subsequent heat treatment or ion implantation.
The actual embossing on the device to be made identifiable is illustrated in Figure 1 b). The metal stamp 1 is pressed onto the surface of the corresponding device 4 until an embossed region 5 is formed under plastic deformation conditions. In this case the result is a general indentation 7 in the region where the grating of the patch is generated. However it is also possible to emboss without having such an overall indentation. A grating 6, which essentially corresponds to the complementary topologically structured surface to the one in the metal stamp is generated on the surface of the device 4.
So in essence the proposed method consists in hammering the desired microstructure into the surface of the device to be securitized by an embossing method using a main die in the form of the metal stamp. This metal stamp can be nano- and/or microstructured with the ionic etching method described above, it may however also itself have been produced in an embossing process.
To be able to hammer a diffractive microstructure with a metal stamp into metal device, e.g. a metallic implant, the following prerequisites should be met:
1. The hardness of the metal stamp should be greater than that of the metal device at the position of the patch.
2. Young's modulus should be as high as possible for both in order to minimize the elastic deformation.
3. The applied stress should be higher than the yield point but lower than the ultimate tensile stress of the compression die. Furthermore, it should be lower than the yield point, if any, and the ultimate tensile stress of the main die.
To be able to nano- and/or microstructure a device based on stainless steel or titanium (alloys) as conventionally used in the field of processes of prosthesis and implants, a main die of hardened steel, for example, is advantageous and an embossing pressure of approximately 0.1-5 kN/mm² is required, preferably in the range 0.2-2 kN/mm². As an alternative to that, the main die may also be made of hardened steel with a coating of tungsten carbide, Si₃N₄ or ZrO₂, for example, which carries the microstructure. The latter embodiment is less expensive because only the coating must be made of the very hard and fracture-resistant material.
Figure 1 c) and d) show that the corresponding process can be used for different medical devices, and the corresponding optical patches can be applied at different places in these devices. In Figure 1 c) the device 4' is a medical distance holder ring.
In figure 1 d) the device is an implant 9. The dental implant 9 comprises an apical threading region 11 and a coronal collar region 10. Typically the apical threading region is provided with a rough surface (by chemical treatment and/or mechanical treatment) which is not suitable for the generation of a grating 6. On the other hand the collar region 10, and in particular axial circumferential surfaces such as the lower abutment surface 12 or the upper terminal surface 13 are suitable for embossing the corresponding optically variable grating patch.
As illustrated in figure 2, the embossing is not limited to flat surfaces such as illustrated in figure 1, the embossing process has the advantage of also being suitable for convex and/or concave surfaces. Essentially any kind of surface can be embossed, all that needs to be taken care of is that the general surface form on the tip 3' of the metal stamp 1 should be complementary to the general surface form of the section of the device 4" where the grating patches to be applied.
Other possibilities of locating a corresponding embossed grating 6 on implants are illustrated in figure 3. On the left side it is illustrated that an embossed grating 6 can be generated on the radial surface 15 of the dental implant which is either converging apically as illustrated in this figure, or also the opposite, if the corresponding surface is converging coronally. On the left side in figure 3 it is illustrated that the corresponding embossed grating 6 can be generated on an abutment 17, specifically on a frustoconical section 18 thereof. While not being illustrated in figure 3 on the right-hand side, it is also possible to generate corresponding patches in a lower apical region of the abutment 17, for example in one of the contact surfaces contacting the implant in use.
Figure 4 illustrates the surface topology on the metal stamp, using the methods as described above a nice grating can be generated if for example a grating period of 1.8 µm is used. The grating has a depth of approximately 360 nm. The grating can be generated over the full patch and it comprises only little lattice imperfections.

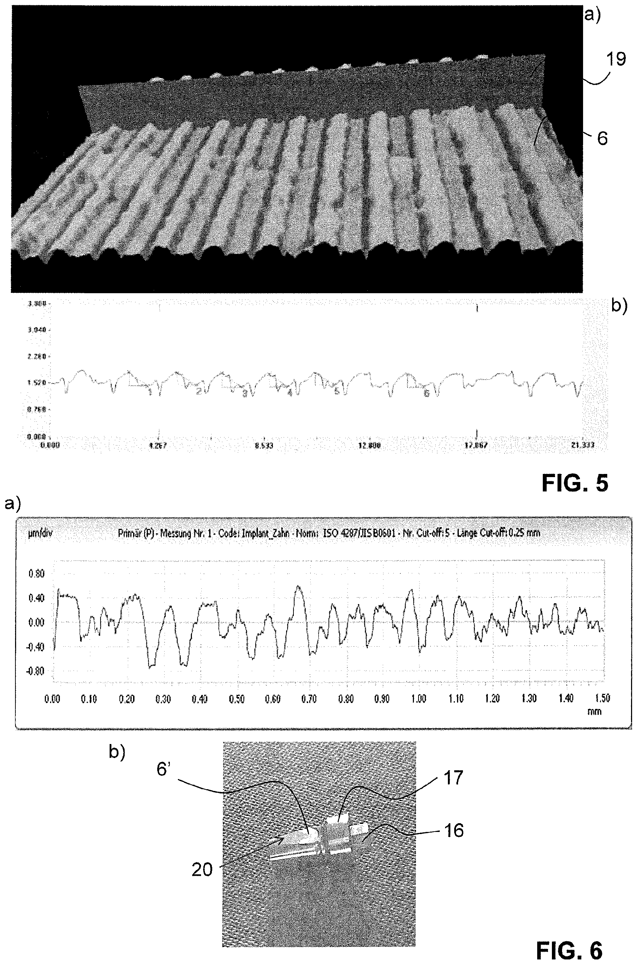

Figure 5 demonstrates that even after having been used repeatedly, the corresponding metal stamp maintains the essential properties of the topologically structured surface. After a series of embossing's in titanium material still the grating has a depth of approximately 360 nm, there is little deposition of titanium on the surface of the metal stamp.

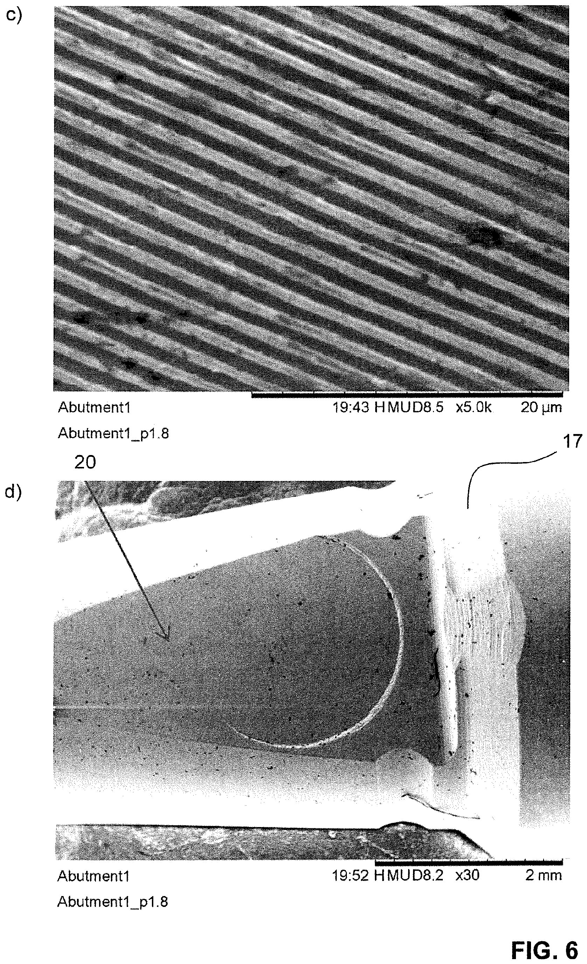

Figure 6 shows a dental abutment 17 in the coronally converging portion thereof there is provided a flattened region 20, and where in this flattened region the optical diffractive element with a grating 6' has been generated. As one can see from a) the corresponding grating has a depth in the range of 250 nm, the proper periodicity, and the structure was generated in an abutment having a roughness value Ra of approximately 0.22 µm. Oppressing force of 4.5 kN was applied at room temperature resulting in a grating as illustrated in c) and d).

**LIST OF REFERENCE SIGNS**

| | | | |
|---|---|---|---|
| 1 | metal stamp | 13 | upper terminal surface on 10 |
| 2 | front portion of 1 | 14 | interface (female) for attaching an implant |
| 3 | topologically structured front | | |
| | surface of 1 | 15 | conical portion of 10 |
| 3' | topologically structured concave front surface of 1 | 16 | interface (male) for attaching the abutment to the implant |
| 3" | topologically structured | 17 | abutment |
| | inclined front surface of 1 | 18 | conical surface of the abutment |
| 4 | object to be provided with a | | |
| | grating | 19 | measurement line |
| 4' | object in the form of a ring | 20 | flattened portion on 18 with optical diffractive element |
| 4" | object with convex surface | | |
| 5 | embossed region in 4 | | |
| 6 | grating embossed in 4 | 30 | master tool |
| 6' | grating in the form of a diffractive optical element | 30a | master 3D topologically structured surface |
| 7 | general indentation | 31 | soft stamp |
| 8 | embossing force | 31a | soft stamp 3D topologically structured surface |
| 9 | dental implant | | |
| 10 | coronal collar region of 9 | 32 | imprint |
| 11 | apical threading region of 9 | 32a | imprint 3D topologically structured surface |
| 12 | lower abutment surface on 10 | | |
| 33 | etch opened imprint 3D topologically structured | | surface |

## Claims

1. Device (4) in the form of a medical implant, a medical prosthesis (9, 17), a medical osteosynthesis device, a hearing aid or a hearing aid housing, in each case at least in part or essentially fully made of metal,
wherein the device (4, 9, 17) comprises at least one nano- and/or microstructure (6) which is directly embossed in an exposed metal surface in the form of a security and/or identification element.

2. Device according to claim 1, wherein the nano- and/or microstructure is an optical diffractive element with a grating (6).

3. Device according to any of the preceding claims, wherein the metal is selected from: steel, preferably stainless steel or implant steel; titanium or a titanium alloy with at least one of zinc, niobium, tantalum, vanadium, aluminium.

4. Device according to any of the preceding claims, wherein the device is a dental implant (9), preferably a dental titanium or dental stainless steel implant.

5. Device according to any of the preceding claims, wherein the period of the nano- and/or microstructure, preferably of the grating (6), is in the range of 0.3-3 µm or in the range of 0.5-2 µm, preferably in the range of 1-1.9 µm or 1.7-1.9 µm and/or wherein the depth of the nano- and/or microstructure, preferably of the grating (6), is in the range of 80-500nm, preferably in the range 200-400 nm, more preferably in the range of 230-300 nm.

6. Device according to any of the preceding claims, wherein the nano- and/or microstructure, preferably the grating (6), is embossed on a ground exposed metal part of the device,
and/or wherein the nano- and/or microstructure, preferably the grating (6), is embossed on an exposed metal part of the device having a surface roughness Ra (as defined according to ISO 4287:1997) of at most 0.8 µm, preferably of at most 0.5 µm or at most 0.3 µm or at most 0.23 µm, preferably in the range of 0.20-0.25 µm.

7. Device according to any of the preceding claims, wherein the nano- and/or microstructure, preferably the grating (6), is embossed using an embossing pressure in the range of 0.1-5 kN/mm², preferably in the range 0.2 - 2 kN/mm² or 0.2-1 kN/mm²
and/or wherein the nano- and/or microstructure, preferably the grating (6), is embossed at a temperature of at most 150°C, preferably at most 100°C, preferably in the range of 10-40°C.

8. Device according to any of the preceding claims, wherein the device is a dental implant(9) or abutment, preferably a dental titanium or dental stainless steel implant or abutment, and wherein preferably the nano- and/or microstructure, preferably the grating (6), is provided in the form of a patch in the coronal collar region (10), preferably on a bright finished metal portion thereof, wherein further preferably the nano- and/or microstructure, preferably the grating (6), is provided on an axial surface (12, 13) covered by an abutment (17) to be mounted on the implant, or is provided on a bright finished exposed metal cylindrical or conical (15) apical portion of the collar region (10).

9. Device according to any of the preceding claims, wherein the device is a dental abutment (17) and wherein preferably the nano- and/or microstructure, preferably the grating (6), is provided on a cylindrical or conical (18) portion of the protruding portion of the abutment.

10. Device according to any of the preceding claims, wherein the nano- and/or microstructure, preferably the grating (6), is provided in the form of a patch with a surface area of at most 5 cm² or at most 5 mm², preferably in the range of 2-4.5 mm²,
and/or wherein the nano- and/or microstructure, preferably the grating (6), is provided such that the tips of the nano- and/or microstructure, preferably the grating (6), are essentially flush with the surface plane defined by the surrounding metal surface.

11. Device according to any of the preceding claims, wherein the nano- and/or microstructure, preferably the grating as an optical diffractive element generates the image of a picture and/or letters and/or numbers and/or pictograms and/or logo.

12. Method for producing a nano- and/or microstructure, preferably an optical diffractive element in the form of a grating (6) on a device according to any of the preceding claims, wherein a metal stamp (1) carrying a topologically structured surface (3) being essentially the negative of the nano- and/or microstructure, preferably the grating (6), to be generated on the device (4) is embossed on an exposed metal surface of the device under plastic deformation conditions such that the topology of the topologically structured surface is imaged on the metal surface of the device (4), wherein preferably the metal stamp has a grating depth in the range of 80-500 nm.

13. Method according to claim 12, wherein the metal stamp (1) at least in the region of the topologically structured surface (3) for embossing, consists of material of a higher hardness than the material of the device (4) in the exposed region to be embossed, wherein preferably the metal stamp is essentially based on hardened steel, preferably with a coating of tungsten carbide, Si3N4 or ZrO2.

14. Method according to any of the preceding claims 12-13, wherein the nano- and/or microstructure, preferably the grating (6), is embossed using an embossing pressure in the range of 0.1-5 kN/mm², preferably in the range 0.2 - 2 or 0.5 - 2 kN/mm²,
and/or wherein the nano- and/or microstructure, preferably the grating (6), is embossed at a temperature of at most 150°C, preferably at most 100°C, preferably in the range of 10-40°C.

## Patentansprüche

1. Vorrichtung (4) in Form eines medizinischen Implantats, einer medizinischen Prothese (9, 17), eines medizinischen Osteosynthesesystems, eines Hörgerätes oder eines Hörgerätegehäuses, jeweils zumindest teilweise oder im wesentlichen vollständig aus Metall,
wobei die Vorrichtung (4, 9, 17) mindestens eine Nano- und/oder Mikrostruktur (6) aufweist, die direkt in eine freiliegende Metalloberfläche in Form eines Sicherheits- und/oder Identifikationselements eingeprägt ist.

2. Vorrichtung nach Anspruch 1, wobei die Nano- und/oder Mikrostruktur ein optisch diffraktives Element mit einem Gitter (6) ist.

3. Vorrichtung nach einem der vorstehenden Ansprüche, wobei das Metall ausgewählt ist aus: Stahl, vorzugsweise rostfreiem Stahl oder Implantatstahl; Titan oder einer Titanlegierung mit mindestens einem der Elemente Zink, Niob, Tantal, Vanadium, Aluminium.

4. Vorrichtung nach einem der vorstehenden Ansprüche, wobei die Vorrichtung ein Dentalimplantat (9) ist, vorzugsweise ein Dentalimplantat aus Titan oder rostfreiem Dentalstahl.

5. Vorrichtung nach einem der vorstehenden Ansprüche, bei der die Periode der Nano- und/oder Mikrostruktur, vorzugsweise des Gitters (6), im Bereich von 0,3-3 µm oder im Bereich von 0,5-2 µm, vorzugsweise im Bereich von 1-1,9 µm oder 1,7-1,9 µm liegt, **dadurch gekennzeichnet, dass** die Vorrichtung ein Zahnimplantat (9), vorzugsweise ein dentales Titan- oder dentales Edelstahlimplantat, ist.
und/oder wobei die Tiefe der Nano- und/oder Mikrostruktur, vorzugsweise des Gitters (6), im Bereich von 80-500 nm, vorzugsweise im Bereich von 200-400 nm, besonders bevorzugt im Bereich von 230-300 nm liegt.

6. Vorrichtung nach einem der vorstehenden Ansprüche, wobei die Nano- und/oder Mikrostruktur, vorzugsweise das Gitter (6), auf einem geschliffenen, freiliegenden Metallteil der Vorrichtung geprägt ist,
und/oder wobei die Nano- und/oder Mikrostruktur, vorzugsweise das Gitter (6), auf einem freiliegenden Metallteil der Vorrichtung mit einer Oberflächenrauhigkeit Ra (wie gemäß ISO 4287:1997 definiert) von höchstens 0,8 µm, vorzugsweise von höchstens 0,5 µm oder höchstens 0,3 µm oder höchstens 0,23 µm, vorzugsweise im Bereich von 0,20-0,25 µm, geprägt ist.

7. Vorrichtung nach einem der vorstehenden Ansprüche, bei der die Nano- und/oder Mikrostruktur, vorzugsweise das Gitter (6), unter Anwendung eines Prägedrucks im Bereich von 0,1-5 kN/mm2, vorzugsweise im Bereich von 0,2 - 2 kN/mm2 oder 0,2-1 kN/mm2, geprägt wird.
und/oder wobei die Nano- und/oder Mikrostruktur, vorzugsweise das Gitter (6), bei einer Temperatur von höchstens 150°C, vorzugsweise höchstens 100°C, vorzugsweise im Bereich von 10-40°C, geprägt wird.

8. Vorrichtung nach einem der vorstehenden Ansprüche, wobei die Vorrichtung ein Dentalimplantat (9) oder ein Abutment, vorzugsweise ein Dentalimplantat aus Titan oder ein Dentalimplantat aus rostfreiem Stahl oder ein Abutment ist, und wobei vorzugsweise die Nano- und/oder Mikrostruktur, vorzugsweise das Gitter (6), in Form eines Flecks im koronalen Kragenbereich (10) vorgesehen ist, vorzugsweise auf einem glänzend bearbeiteten Metallteil davon, wobei ferner vorzugsweise die Nano- und/oder Mikrostruktur, vorzugsweise das Gitter (6), auf einer axialen Oberfläche (12, 13) vorgesehen ist, die von einem auf dem Implantat anzubringenden Widerlager (17) bedeckt ist, oder auf einem glänzend bearbeiteten, freiliegenden, zylindrischen oder konischen (15) apikalen Teil des Kragenbereichs (10) aus Metall vorgesehen ist.

9. Vorrichtung nach einem der vorstehenden Ansprüche, wobei die Vorrichtung ein Dentalabutment (17) ist und wobei vorzugsweise die Nano- und/oder Mikrostruktur, vorzugsweise das Gitter (6), auf einem zylindrischen oder konischen (18) Abschnitt des vorstehenden Abschnitts des Abutments vorgesehen ist.

10. Vorrichtung nach einem der vorstehenden Ansprüche, wobei die Nano- und/oder Mikrostruktur, vorzugsweise das Gitter (6), in Form eines Flecks mit einer Oberfläche von höchstens 5 cm² oder höchstens 5 mm2, vorzugsweise im Bereich von 2-4,5 mm², vorgesehen ist,
und/oder wobei die Nano- und/oder Mikrostruktur, vorzugsweise das Gitter (6), so vorgesehen ist, dass die Spitzen der Nano- und/oder Mikrostruktur, vorzugsweise des Gitters (6), im Wesentlichen bündig mit der durch die umgebende Metalloberfläche definierten Oberflächenebene sind.

11. Vorrichtung nach einem der vorstehenden Ansprüche, wobei die Nano- und/oder Mikrostruktur, vorzugsweise das Gitter als optisch diffraktives Element das Bild eines Bildes und/oder Buchstaben und/oder Zahlen und/oder Piktogramme und/oder Logo erzeugt.

12. Verfahren zur Herstellung einer Nano- und/oder Mikrostruktur, vorzugsweise eines optischen Beugungselements in Form eines Gitters (6) auf einer Vorrichtung nach einem der vorstehenden Ansprüche, wobei ein Metallstempel (1), der eine topologisch strukturierte Oberfläche (3) trägt, im Wesentlichen das Negativ der Nano- und/oder Mikrostruktur, vorzugsweise des Gitters (6), ist, auf der Vorrichtung (4) zu erzeugen ist, auf einer freiliegenden Metalloberfläche der Vorrichtung unter plastischen Verformungsbedingungen geprägt wird, so dass die Topologie der topologisch strukturierten Oberfläche auf der Metalloberfläche der Vorrichtung (4) abgebildet wird, wobei vorzugsweise der Metallstempel eine Gittertiefe im Bereich von 80-500 nm aufweist.

13. Verfahren nach Anspruch 12, wobei der Metallstempel (1) zumindest im Bereich der topologisch strukturierten Oberfläche (3) für die Prägung aus Material mit einer höheren Härte als das Material der Vorrichtung (4) in dem freiliegenden, zu prägenden Bereich besteht, wobei vorzugsweise der Metallstempel im Wesentlichen auf gehärtetem Stahl basiert, vorzugsweise mit einer Beschichtung aus Wolframkarbid, Si3N4 oder ZrO2.

14. Verfahren nach einem der vorhergehenden Ansprüche 12-13, wobei die Nano- und/oder Mikrostruktur, vorzugsweise das Gitter (6), unter Verwendung eines Prägedrucks im Bereich von 0,1-5 kN/mm2, vorzugsweise im Bereich 0,2 - 2 oder 0,5 - 2 kN/mm2, geprägt wird,
und/oder wobei die Nano- und/oder Mikrostruktur, vorzugsweise das Gitter (6), bei einer Temperatur von höchstens 150°C, vorzugsweise höchstens 100°C, vorzugsweise im Bereich von 10-40°C, geprägt wird.

## Revendications

1. Dispositif (4) sous forme d'implant médical, de prothèse médicale (9, 17), de dispositif médical d'ostéosynthèse, de prothèse auditive ou de boîtier de prothèse auditive, dans chaque cas au moins en partie ou essentiellement en totalité en métal,
dans lequel le dispositif (4, 9, 17) comprend au moins une nano- et/ou microstructure (6) qui est directement estampée dans une surface métallique exposée sous la forme d'un élément de sécurité et/ou d'identification.

2. Dispositif selon la revendication 1, dans lequel la nano- et/ou microstructure est un élément optique diffractif avec un réseau (6).

3. Dispositif selon l'une des revendications précédentes, dans lequel le métal est choisi parmi : l'acier, de préférence l'acier inoxydable ou l'acier pour implants ; le titane ou un alliage de titane avec au moins l'un des éléments suivants : zinc, niobium, tantale, vanadium, aluminium.

4. Dispositif selon l'une des revendications précédentes, dans lequel le dispositif est un implant dentaire (9), de préférence un implant dentaire en titane ou en acier inoxydable.

5. Dispositif selon l'une des revendications précédentes, dans lequel la période de la nano- et/ou microstructure, de préférence du réseau (6), est comprise entre 0,3 et 3 µm ou entre 0,5 et 2 µm, de préférence entre 1 et 1,9 µm ou entre 1,7 et 1,9 µm et/ou dans lequel la profondeur de la nano- et/ou microstructure, de préférence du réseau (6), est comprise entre 80 et 500 nm, de préférence entre 200 et 400 nm, et plus préférablement entre 230 et 300 nm.

6. Dispositif selon l'une des revendications précédentes, dans lequel la nano- et/ou la microstructure, de préférence le réseau (6), est estampée sur une partie métallique du dispositif exposée au sol,
et/ou dans lequel la nano- et/ou microstructure, de préférence le réseau (6), est estampée sur une partie métallique exposée du dispositif ayant une rugosité de surface
Ra (telle que définie selon la norme ISO 4287:1997) d'au plus 0,8 µm, de préférence d'au plus 0,5 µm ou d'au plus 0,3 µm ou d'au plus 0,23 µm, de préférence dans la plage de 0,20-0,25 µm.

7. Dispositif selon l'une des revendications précédentes, dans lequel la nano- et/ou microstructure, de préférence le réseau (6), est gaufrée en utilisant une pression de gaufrage dans la gamme de 0,1-5 kN/mm2, de préférence dans la gamme de 0,2-2 kN/mm2 ou 0,2-1 kN/mm2
et/ou dans lequel la nano- et/ou microstructure, de préférence le réseau (6), est gaufrée à une température de 150°C au maximum, de préférence de 100°C au maximum, de préférence dans la gamme de 10 à 40°C.

8. Dispositif selon l'une des revendications précédentes, dans lequel le dispositif est un implant dentaire (9) ou un pilier, de préférence un implant ou un pilier dentaire en titane ou en acier inoxydable, et dans lequel de préférence la nano- et/ou la microstructure, de préférence le réseau (6), est prévue sous la forme d'un patch dans la région du collet coronal (10), de préférence sur une partie métallique à finition brillante de celui-ci, dans lequel en outre, de préférence, la nano- et/ou microstructure, de préférence le réseau (6), est prévue sur une surface axiale (12, 13) recouverte d'un pilier (17) à monter sur l'implant, ou est prévue sur une partie apicale cylindrique ou conique (15) en métal exposé à finition brillante de la région du col (10).

9. Dispositif selon l'une des revendications précédentes, dans lequel le dispositif est un pilier dentaire (17) et dans lequel de préférence la nano- et/ou la microstructure, de préférence le réseau (6), est prévue sur une partie cylindrique ou conique (18) de la partie saillante du pilier.

10. Dispositif selon l'une des revendications précédentes, dans lequel la nano- et/ou la microstructure, de préférence le réseau (6), est fournie sous la forme d'un patch avec une surface d'au plus 5 cm² ou d'au plus 5 mm2, de préférence dans la gamme de 2-4,5 mm²,
et/ou dans lequel la nano- et/ou microstructure, de préférence le réseau (6), est prévue de telle sorte que les pointes de la nano- et/ou microstructure, de préférence le réseau (6), soient essentiellement à fleur du plan de surface défini par la surface métallique environnante.

11. Dispositif selon l'une des revendications précédentes, dans lequel la nano- et/ou microstructure, de préférence le réseau en tant qu'élément diffractif optique, génère l'image d'une image et/ou de lettres et/ou de chiffres et/ou de pictogrammes et/ou d'un logo.

12. Procédé de production d'une nano- et/ou microstructure, de préférence d'un élément diffractif optique sous la forme d'un réseau (6) sur un dispositif selon l'une des revendications précédentes, dans lequel un poinçon métallique (1) portant une surface topologiquement structurée (3) est essentiellement le négatif de la nano- et/ou microstructure, de préférence du réseau (6), à générer sur le dispositif (4) est estampé sur une surface métallique exposée du dispositif dans des conditions de déformation plastique de telle sorte que la topologie de la surface topologiquement structurée est représentée sur la surface métallique du dispositif (4), dans lequel de préférence l'estampe métallique a une profondeur de réseau dans la plage de 80-500 nm.

13. Procédé selon la revendication 12, le poinçon métallique (1) étant constitué, au moins dans la zone de la surface topologiquement structurée (3) à estamper, d'un matériau d'une dureté supérieure à celle du matériau du dispositif (4) dans la zone exposée à estamper, le poinçon métallique étant de préférence essentiellement à base d'acier trempé, de préférence avec un revêtement en carbure de tungstène, Si3N4 ou ZrO2.

14. Procédé selon l'une des revendications précédentes 12-13, dans lequel la nano- et/ou la microstructure, de préférence le réseau (6), est estampée en utilisant une pression d'estampage dans la plage de 0,1-5 kN/mm2, de préférence dans la plage de 0,2 - 2 ou 0,5 - 2 kN/mm2,
et/ou dans lequel la nano- et/ou microstructure, de préférence le réseau (6), est gaufrée à une température de 150°C au maximum, de préférence de 100°C au maximum, de préférence dans la gamme de 10 à 40°C.
